# FASCICULE DE BREVET EUROPEEN

(11) **EP 2 018 544 B1**
(45) Date de publication et mention de la délivrance du brevet: **10.08.2016**
(21) Numéro de dépôt: 07766020.7
(22) Date de dépôt: 09.05.2007
(51) Int. Cl.: G01N 22/04, G01N 33/38

(54) **METHODE DE DETECTION DES DEFAUTS LOCALISES PRESENTS DANS UN MATELAS DE FIBRES MINERALES**
VERFAHREN FÜR DEN NACHWEIS ÖRTLICHER DEFEKTE IN EINER MINERALFASERMATTE
METHOD FOR DETECTING LOCALIZED DEFECTS PRESENT IN A MINERAL FIBER MAT

(30) Priorité: 10.05.2006 FR 0651683
(43) Date de publication de la demande: 28.01.2009
(73) Titulaire: SAINT-GOBAIN ISOVER, 92400 Courbevoie (FR)
(72) Inventeur: ELLISON, Christopher, 60140 Liancourt (FR)
(74) Mandataire: Lucas, Francois
(86) Numéro de dépôt international: PCT/FR2007/051240
(87) Numéro de publication internationale: WO 2007/128942

(56) Documents cités:
- WO-A2-2006/023137
- DE-A1- 10 053 112
- GB-A- 2 300 483
- US-A- 4 203 155

## Description

L'invention se rapporte au domaine des matériaux constitués par un matelas de fibres minérales, notamment de fibres de roche ou des fibres de verre. Plus particulièrement, la présente invention se rapporte à la détection de défauts localisés qui peuvent être présents au sein du matelas fibreux notamment dans ou à l'issue d'un processus de fabrication de produits destinés à l'isolation acoustique et/ou thermique ou encore utilisés comme support ou substrat de croissance des plants.

Les matériaux d'isolation commercialisés à l'heure actuelle sont constitués d'un matelas ou feutre de fibres minérales, telles que des fibres de verre ou des fibres de roche liées par un liant organique.

Le procédé de fabrication de ces matelas de fibres est bien connu et comprend typiquement, par exemple dans le cas d'un panneau de laine de roche, la succession des étapes suivantes :
- la fusion de la roche généralement dans un cubilot à une température de l'ordre de 1500°C,
- le fibrage, c'est-à-dire l'obtention de fibre de roche par introduction du matériau en fusion dans un dispositif de centrifugation externe connu à cet effet et le plus souvent appelé rotative, par exemple tel que décrit dans le brevet EP 119 124,
- la pulvérisation d'une composition d'encollage comprenant un liant généralement thermo-durcissable en solution aqueuse sur les fibres nouvellement formées,
- la réception des fibres imprégnées du liant dans une chambre de réception comprenant à l'opposé du dispositif de fibrage un convoyeur muni dans sa partie inférieure de caissons d'aspirations maintenus en dépression,
- une cuisson dans une étuve ou un four à une température et pendant une durée suffisante pour permettre le durcissement et la réticulation du liant et l'élimination de l'eau résiduelle,
- une découpe longitudinale des bords non réguliers, et éventuellement du centre, du matelas de fibres continu en longueur ainsi obtenu, au moyen de scies disposées le long du convoyeur,
- une découpe du matelas dans un sens transversal et éventuellement dans le sens de l'épaisseur (refente), de manière à obtenir des blocs qui pourront ensuite être soit disposés en plaques ou en rouleau, généralement au moyen d'une scie ou d'un massicot,
- un stockage sur palette des plaques ou des rouleaux, avant expédition.

Le procédé de fabrication de laine de verre se décompose de façon similaire à la différence près qu'on utilise un four pour la fusion et un dispositif de centrifugation interne pour le fibrage par exemple tel que décrit dans EP91866.

Dans le cadre du contrôle du procédé de fabrication tel qu'il vient d'être décrit, il est nécessaire d'effectuer en continu des procédures de contrôle, sur au moins une partie et de préférence sur la totalité de la production, pour garantir une bonne qualité du matelas. De tels contrôles portent notamment sur le degré de cuisson du liant à la sortie de l'étuve, au travers par exemple d'une mesure de la teneur en eau résiduelle du matelas de fibres et/ou d'une estimation du degré de polymérisation du liant.

Il est bien évident que ces procédures de contrôle ne doivent cependant pas gêner le bon déroulement des différentes étapes du procédé. En particulier, selon certaines caractéristiques essentielles recherchées, de telles procédures doivent pouvoir être mises en oeuvre efficacement et facilement sur une ligne de production préexistante, sans entraîner de surcoûts importants. Selon un autre paramètre important, le contrôle du procédé doit être réactif, c'est-à-dire que la mesure doit pouvoir être effectuée dans un temps suffisamment court pour permettre une action rapide de l'opérateur et éviter ainsi la mise au rebut et la perte d'une partie de la production, ou au moins pour limiter la perte subie au minimum.

De préférence, la méthode doit être du type non invasif, c'est-à-dire qu'elle ne nécessite pas l'incorporation d'éléments étrangers dans le matelas de fibres. Des méthodes invasives connues sont par exemple les méthodes impliquant l'utilisation d'une mesure de température par des thermocouples embarqués dans le produit, le cas échéant avec un enregistreur, tel que par exemple décrit dans la demande WO2006/017106. On connaît aussi des méthodes impliquant l'utilisation de réactifs chimiques tels qu'un indicateur de pH. En général de telles méthodes sont manuelles et nécessitent le prélèvement d'un échantillon sur la ligne, analysé dans un deuxième temps en laboratoire.

Selon une autre voie, la demande de brevet WO 2006/023137 décrit par exemple une méthode de contrôle du procédé basée sur une mesure par des moyens spectroscopiques de l'humidité résiduelle présente dans le matelas de fibres imprégné du liant, avant le traitement thermique de réticulation du liant dans l'étuve.
Plus précisément, la méthode comprend une mesure du taux d'humidité global résiduel, c'est-à-dire de la quantité globale d'eau présente dans le matelas en sortie de la chambre de réception des fibres. Par comparaison de ladite valeur du taux d'humidité résiduel avec une valeur de référence, l'ajustement d'au moins un paramètre permet le contrôle en continu du procédé. Selon ce document, ce paramètre peut être choisi parmi la quantité d'eau initialement mélangée au liant, la température de la chambre de collecte ou la valeur de la dépression appliquée sur les caissons d'aspirations.

Avec les résines formo-phénoliques usuelles qui jaunissent à la cuisson, ce contrôle peut être fait visuellement par appréciation de la couleur du produit par un opérateur. Cette méthode est néanmoins approximative en ce qui concerne notamment des défauts plus localisés et elle ne permet pas en outre de détecter les défauts dans l'épaisseur du produit.

Il est à noter que l'importance et la nécessité d'une méthode de contrôle efficace dans les procédés de fabrication des matelas de fibres minérales sont encore renforcées à l'heure actuelle en raison de la volonté de développer des systèmes liants alternatifs en substitution des résines formo-phénoliques, qui permettraient de réduire les risques de dégagement de formaldéhyde lors de la cuisson du liant.

Plus particulièrement, on étudie actuellement des liants de nature chimique différente, notamment obtenus à partir de polymères polycarboxyliques et de polyols tels que les résines acryliques, par exemple tels que décrits dans la demande WO 2006/023137. A la différence des résines formo-phénoliques, ces liants ne présentent pas de modifications d'aspect ou de couleur caractéristiques à la cuisson. En outre, pour éviter une polymérisation et une prise trop précoce de tels liants et pour en diminuer la viscosité, il est nécessaire d'augmenter sensiblement, par rapport aux liants formo-phénoliques, la proportion d'eau présente dans la solution pulvérisée sur les fibres néoformées, ce qui entraîne des difficultés accrues au niveau de l'élimination de l'eau résiduelle potentiellement présente en sortie de ligne sur le produit fini, et rend ainsi plus encore indispensable la présence d'outils de contrôle en continu de la production, répondant si possible à l'ensemble des caractéristiques précédemment décrites.

Il n'existe cependant à l'heure actuelle que très peu de systèmes permettant de répondre à l'ensemble de ces exigences et de contrôler efficacement en continu la qualité du matelas de fibres, que ce soit en sortie de ligne ou en reprise, c'est-à-dire après la production elle même.

Parmi quelques méthodes connues, on peut citer celles incluant des dispositifs générant des radiations, tels que les sondes ou jauges gamma telles que décrites dans le brevet EP118369 ou les rayons X mais de tels appareils, outre qu'ils sont relativement coûteux, nécessitent de prendre des précautions d'utilisation extrêmement draconiennes pour la sécurité du personnel, avec notamment l'obligation de prévoir un périmètre important de sécurité autour de la source.

D'autres méthodes connues sont par exemple les méthodes spectroscopiques, basées sur un rayonnement infrarouge (IR) pour mesurer le taux d'humidité résiduel global du matelas de fibres. Cependant, le pouvoir pénétrant des IR étant relativement faible, l'analyse est limité à la partie la plus externe du matelas et le taux d'humidité au coeur du matériau, qui peut être relativement important, ne peut être déterminé par cette méthode.

D'un autre coté, l'ensemble des méthodes non invasives actuellement utilisées ou connues pour le contrôle de la qualité et de l'homogénéité d'un matelas de fibres minérales liées par un liant ne rendent compte que d'une valeur moyenne à un instant t, au sein d'une portion du matériau, du paramètre de contrôle, en particulier de la teneur en eau globale présente au sein de ladite portion.
En particulier, de telles méthodes ne sont pas assez discriminantes pour distinguer des défauts localisés, c'est-à-dire des défauts présents uniquement en certains points très localisés du feutre. On peut classer ces défauts en deux catégories principales :
1°) les défauts « humides » ou points humides, qui correspondent à des emplacements très localisés sur le feutre. Ces défauts apparaissent notamment à des endroits où, lors de l'étape de fibrage, un amas de densité supérieure de fibres, d'eau et de résine (liant) se forme. Lors de l'étape de séchage par aspiration, l'air chaud traversant le matelas a alors tendance à contourner ce point de densité supérieure, au niveau duquel subsiste donc une forte concentration de résine. De tels points humides entraînent un dégagement progressif de formaldéhyde, jusque très longtemps après la fabrication. De tels dégagements ne doivent bien entendu n'être qu'exceptionnels voire inexistants, dans le produit fini, notamment au regard de la sévérisation des normes actuelles ou à venir.
   En outre, les points humides sont très préjudiciables à certaines utilisations particulières des matelas fibreux et dans certains cas extrêmes les empêchent. Par exemple, la présence de tels défauts dans un matelas fibreux utilisé comme support ou substrat de croissance des plants entraîne la mort des végétaux disposés à leur proximité.
   Au final, dans de nombreuses applications, un recuit de l'ensemble de la production, très coûteux, s'avère nécessaire pour être certain de l'absence de tels points humides.
2°) les défauts du type « points chauds », qui correspondent à des zones de forte densité en roche ou en verre. De tels défauts sont issus du processus de fibrage, et apparaissent notamment en raison d'instabilités ou d'accumulations de projections d'infibrés. Ils se caractérisent par des amas très localisés à l'intérieur du matelas fibreux. Ces amas peuvent soit refroidir au sein du feutre et donner naissance ainsi à des zones extrêmement denses et dures sur lesquels les dispositifs de coupe (scie, massicot) peuvent ensuite venir de briser ou se fendre, soit continuer une fusion lente qui peut dans les cas extrêmes aboutir à la prise en feu de l'ensemble du produit, par exemple dans des zones de stockage.
   Bien entendu, ce qui vient d'être décrit ne constitue que quelques exemples de défauts localisés au sens de la présente invention qui n'est évidemment pas limitée à la détection de ces seuls défauts. De manière générale, tout défaut contribuant à variation locale telle une augmentation (ou une diminution) locale de la concentration en fibres et/ou en eau et/ou en liant au sein du matelas doit être considéré comme compris dans la présente invention. En particulier, la détection d'un défaut constitué par une augmentation locale de la densité ou grammage du matelas de fibre est comprise dans le cadre de la présente invention.

Plus particulièrement, la présente invention se rapporte à une méthode selon la revendication 1, permettant de résoudre l'ensemble des problèmes précédemment exposés, permettant notamment la détection de défauts localisés, par exemple mais non limitativement du type points chauds ou points humides, présents dans un matelas de fibres minérales liés par un liant, comprenant l'utilisation d'un rayonnement micro-ondes de fréquence comprise entre 1 et 50 GHz, de préférence entre 5 et 10 GHz, la puissance totale de l'onde électromagnétique émise étant comprise entre 0,1 et 5 Watts, de préférence de l'ordre de ou inférieur à 1 Watt, voire même l'ordre de ou inférieur à 0,5 Watt.

De façon étonnante et inattendue, les expériences menées par le demandeur, dont plusieurs exemples seront donnés dans la suite de la description, ont montré que d'aussi faibles puissances étaient cependant suffisantes pour l'analyse d'un matelas fibreux dans toute son épaisseur. A titre d'exemple, on a pu visualiser, suivant la présente méthode, la présence des défauts localisés précédemment décrits dans des feutres dont l'épaisseur est comprise entre 30 et 400 mm, voire même supérieure et dont la densité est comprise entre 6 et 220 kg/m³, voire supérieure. Typiquement, la densité de la laine de verre peut par exemple être comprise entre 6 et 140 kg/m³ et la densité de la laine de roche peut être comprise entre 20 et 220 kg/m³.

Bien entendu, il est possible selon l'invention de faire varier la puissance des micro-ondes incidents suivant l'un ou l'autre de ces paramètres. Cependant, conformément à un des aspects essentiels de l'invention, cette puissance reste toujours dans des valeurs de l'ordre du Watt. Leur mise en oeuvre, au contraire de la plupart des méthodes de contrôle non invasives actuellement utilisées, ne présente donc aucun danger pour le personnel. En particulier, la présente méthode ne nécessite pas de prendre des précautions d'utilisation pour la sécurité du personnel, ni l'obligation de prévoir un périmètre important de sécurité autour du dispositif de mesure, notamment au sens de la directive européenne 2004/40/CE du parlement européen, relative aux prescriptions minimales d'exposition des travailleurs aux champ électromagnétiques. A titre de comparaison, on a pu mesurer que la puissance émise autour de l'antenne d'un téléphone portable est de l'ordre de 2 Watts.

Par exemple, la présence desdits défauts est caractérisée par une mesure du décalage de la phase du rayonnement micro-ondes et/ou de la modification de l'amplitude dudit rayonnement, lorsque celui-ci traverse le matelas de fibres minérales.

Selon l'invention, le rayonnement micro-onde incident est mis en oeuvre au moyen d'une série ou d'un réseau de dispositifs émetteurs des micro-ondes, disposés sensiblement transversalement en regard d'une face du matelas et orientés en direction dudit matelas. Le signal, après traversée dudit matelas, est recueilli par une série ou un réseau de sondes réceptrices ou de capteurs disposés sensiblement transversalement en regard de la face opposée du matelas. De préférence, le réseau de capteurs est placé au droit du réseau des émetteurs.

Avantageusement, le matelas défile entre le réseau de dispositifs émetteurs et le réseau de sondes réceptrices, la détection desdits défauts étant effectuée en continu sur toute la largeur du matelas de fibres.

Typiquement, le matelas de fibres défile sur un convoyeur à rouleau, les dispositifs émetteurs et les capteurs étant disposés respectivement au dessus et en dessous du convoyeur.

L'écartement entre les capteurs peut être choisi en fonction de la taille desdits défauts, l'écartement entre les capteurs étant par exemple compris entre 1 et 100 mm, de préférence entre 5 et 20 mm.

La méthode selon l'invention est mise en oeuvre par un dispositif permettant la mise en oeuvre de la méthode de détection précédemment décrite, comprenant des moyens de génération d'un rayonnement micro-ondes de fréquence comprise entre 1 et 50 GHz, de préférence entre 5 et 10 GHz, la puissance totale de l'onde électromagnétique émise étant comprise entre 0,1 et 5 Watts, de préférence de l'ordre de ou inférieur à 1 Watt et des moyens de détection de l'onde électromagnétique, après traversée du matelas de fibres.

Les moyens de génération comprennent par exemple une série ou un réseau de dispositifs émetteurs de micro-ondes disposés de manière à pouvoir être orientés transversalement selon une face du matelas et en direction dudit matelas. Les moyens de détection comprennent par exemple une série ou un réseau de capteurs disposés transversalement en regard de la face opposée du matelas de fibres. De préférence, les moyens de génération sont placés au droit des moyens de détection.

Selon un mode de réalisation préféré, le dispositif comprend en outre des moyens de traitement et de visualisation du signal.

L'invention se rapporte également à une installation selon la revendication 13 pour la fabrication d'un matelas continu de fibres minérales liées par un liant, du type laine de roche ou laine de verre, comprenant des moyens de fibrage des fibres minérales, des moyens de synthèse et d'aspersion du liant, des moyens de collecte, de convoyage et de réticulation des fibres, ladite installation comprenant en outre un dispositif de détection tel que précédemment décrit, disposé à la sortie des moyens de réticulation.

Avantageusement, le dispositif de détection est alors soit couplé à des moyens de régulation d'au moins un paramètre choisi dans le groupe constitué par la composition du liant, la force de l'aspiration, la température de réticulation des fibres, le temps de séjour dans les moyens de réticulation, soit couplé à des moyens de régulation d'un organe disposé en aval du dispositif d'analyse micro-ondes, ledit organe étant configuré pour isoler et/ou déclasser les zones du matelas incluant les défauts ou pour effectuer un marquage très précis desdites zones, par exemple en vue d'une découpe ou d'un tri ultérieur.

L'invention dans ses détails sera mieux comprise à la lecture de la description qui suit d'une mise en oeuvre du présent dispositif au sein d'une ligne de fibrage d'un matelas de laine minérale. Dans le cadre de la présente invention, d'autres modes de réalisation sont bien entendu possibles, la description qui suit étant uniquement fournie à titre d'illustration et ne devant être considérée comme limitative sous aucun de ses aspects décrits.
La figure 1 représente une vue synoptique d'un schéma de fabrication d'une laine minérale, incorporant la présente invention.
La figure 2 est une vue schématique plus détaillée d'un exemple de réalisation du dispositif selon l'invention.

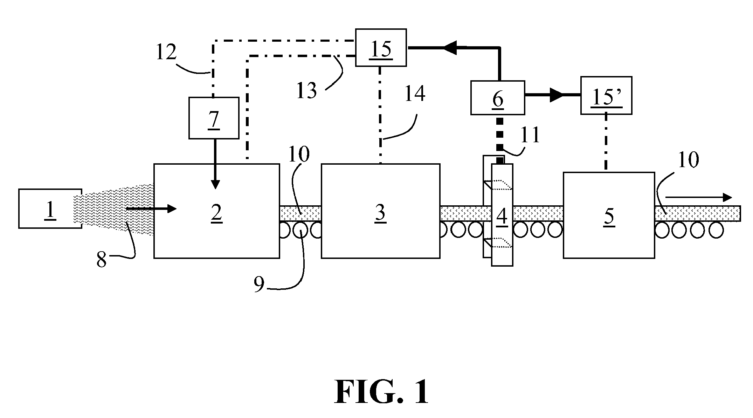

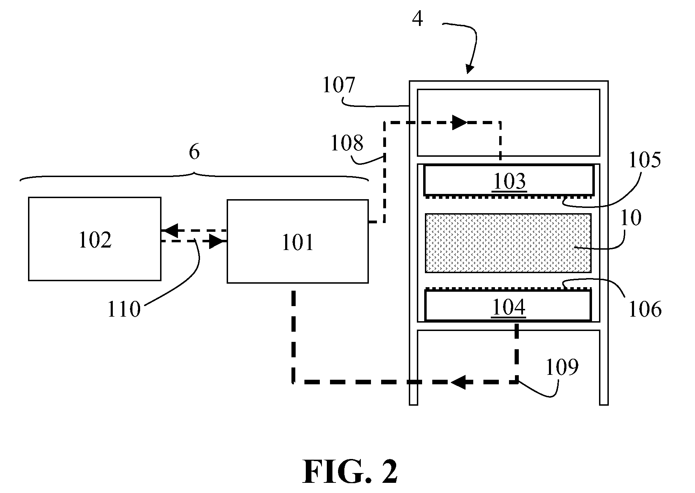

Sur la figure 1, est représentée schématiquement une installation de fibrage tel que précédemment décrite. L'installation comprend un dispositif 1 de technologie connue, permettant le fibrage de la roche ou du verre. Selon un procédé bien connu, les fibres 8 sont collectées sous la forme d'un matelas dans une chambre de réception 2 telle que déjà décrite, en même temps qu'une composition d'encollage est pulvérisée sur les fibres néoformées 8 par des moyens dédiés 7 de mélange et d'injection des différents constituants (résine, eau, adjuvants, etc.).
Le matelas de fibres 10 est ensuite calibré et envoyé grâce à des moyens de convoyage 9 dans une étuve 3, dont la température est par exemple proche de 400°C. La montée en température et le temps de séjour du matelas fibreux dans l'étuve sont ajustés pour permettre la cuisson du liant et l'élimination de l'eau. En sortie de l'étuve 3, le matelas continu de fibres est alors soumis à un contrôle en continu par le dispositif à micro-ondes basse puissance selon l'invention. Le dispositif 4 détecte alors selon l'invention les éventuels défauts localisés présents sur toute la longueur et dans toute l'épaisseur du matelas de fibres. Les informations collectées 11 sont transmises à un dispositif 6 permettant le traitement et l'amplification du signal de sortie et éventuellement sa visualisation. Un mode de réalisation et de fonctionnement du dispositif 6 est illustré par la figure 2.

Après traitement par le dispositif 6, si le signal 11 indique la présence de défauts localisés, l'information est transmise à un dispositif de contrôle 15 qui est alors susceptible de modifier en continu et dans un délai très bref au moins un des paramètres agissant sur une étape du procédé de fabrication, par exemple via des lignes de commande 12, 13, 14. Ledit paramètre peut être par exemple, mais non limitativement, la quantité de liant injectée, la composition initiale du mélange et notamment la quantité d'eau présente avec la résine (ligne de commande 12), la température de l'étuve (ligne 14), la force de l'aspiration des caissons (ligne 13) etc...

Selon une autre réalisation possible de l'invention, qui peut être couplée avec la précédente, un dispositif de contrôle 15' gère un organe 5 disposé en aval du dispositif d'analyse micro-ondes 4. Cet organe peut avoir par exemple pour rôle d'isoler et/ou de déclasser les zones du matelas incluant les défauts ou d'effectuer un marquage très précis desdites zones, par exemple en vue d'une découpe ou d'un tri ultérieur. Une telle réalisation permet ainsi d'une part d'assurer une qualité de production constante et d'autre part de restreindre au strict minimum la fraction de la production devant être placée au rebut ou recyclée. Bien entendu, d'autres réalisations et variantes du procédé de contrôle de la fabrication sont possibles et doivent être compris dans le cadre de la présente invention.
En particulier, le dispositif selon l'invention peut également être utilisé pour le contrôle de la qualité de la laine minérale et son tri pour toute application « hors ligne de production ». Par exemple, il est possible, sans sortir du cadre de l'invention, qu'un tri plus fin soit opéré, en usine ou éventuellement chez un client en fonction de ses besoins, dans un atelier de découpe ou transformation utilisant le présent dispositif. Ceci permet notamment d'éviter et de garantir que des défauts, habituellement acceptables pour une application ordinaire, n'entrent pas dans la composition du matelas de fibres, lorsqu'une application plus spécifique est envisagée (par exemple mais non limitativement la culture de plants).

La figure 2 représente schématiquement le fonctionnement des dispositifs 4 de détection et 6 de génération/traitement du signal. Sur la figure 2, le dispositif 6 comprend une unité 101 de génération, de réception et d'amplification des radiofréquences RF de technologie connue et une unité 102 de traitement et de visualisation du signal, incluant une interface 110 vers un micro-ordinateur sur lequel peuvent être visualisés les défauts présents sur toute la largeur et dans toute l'épaisseur du matelas 10. Le défilement du matelas à travers le dispositif de détection 4 et le traitement immédiat du signal obtenu par le dispositif 6 permettent avantageusement de caractériser, quasiment en temps réel sur la ligne, les défauts présents sur toute la longueur du matelas. L'unité RF 101 génère un signal radiofréquence approprié qui alimente une antenne 103 par l'intermédiaire d'un câble radiofréquence 108. De façon connue, le signal incident est par exemple divisé par une série de diviseurs, de telle manière qu'un signal ou rayonnement micro-onde présentant un front d'onde uniforme et de longueur sensiblement égale à la largeur du matelas soit généré, au niveau de l'antenne 103, en direction du matelas 10, par une série d'émetteurs 105. Ces émetteurs sont par exemple disposés transversalement par exemple selon N réseaux de n plages ou patchs espacés régulièrement, par exemple de 10 mm, au niveau de la partie inférieure de l'antenne. La méthode consiste en une mesure sur toute la largeur du matelas de fibres du coefficient de transmission à travers celui-ci. On ne sortirait cependant pas du cadre de l'invention si la mesure était effectuée en réflexion, après que le rayonnement micro-onde incident ait traversé le matelas de fibres.

Selon l'invention, la puissance totale générée par la totalité des émetteurs 105 est de l'ordre du watt, voire même inférieure. Le rayonnement, après traversée du matelas 10, est recueilli, au niveau de la partie réceptrice 104, par au moins un réseau de capteurs 106 disposés transversalement en regard de la face opposée du matelas 10. La partie collectrice comprend typiquement une rétine constituée d'un nombre approprié de sondes incorporant des éléments actifs, réparties par exemple en N réseaux de n plages ou patchs, au sein d'une antenne collectrice. Le nombre de sondes utilisées et l'espacement entre deux sondes successives est fonction de la largeur du matelas à étudier et de la résolution souhaitée, notamment au regard de la taille des défauts recherchés. Selon le principe de l'invention, la présence d'un défaut localisé selon l'invention au sein du matelas de fibres traversé par l'onde micro-onde basse puissance incidente, entraîne une perturbation électromagnétique de celle-ci et une modulation du signal. En sortie de la partie réceptrice 104, le signal micro-onde modulé, dont l'amplitude et la phase sont fonction de la nature et de la taille des défauts rencontrés par le rayonnement, est recueilli.

Le signal modulé est ensuite transmis et traité selon des techniques connues, par exemple telles que décrites par la suite, par les unités 101 et 102 via un câble radiofréquence 109. Les parties émettrices et réceptrices des micro-ondes sont positionnées sur un bâti 107, lui-même disposé autour du dispositif de convoyage à rouleaux 9, tel qu'indiqué sur la figure 1.

Des exemples de technologie micro-onde fonctionnant selon les principes précédemment décrits et pouvant être utilisés selon l'invention sont notamment décrites dans les publications scientifiques « Materials Research Society Symposia Proceedings, année 1991, volume 189, pages 49-53 » ou « International Microwave Symposium Digest, année 1990, volume 3, pages 1133-1136 », auxquelles on se référera pour les détails de la mise en oeuvre.

Selon l'invention, la détection et le traitement du signal peuvent être réalisés selon toutes les techniques d'analyse et de traitement de signaux connus de l'art, par exemple par seuillage, par moyennage géométrique et/ou arithmétique, ou par l'utilisation d'autres algorithmes de traitement et filtrage des signaux, ainsi que par l'analyse des franges de diffraction entre signaux, notamment pour amplifier la détection. Selon une possibilité avantageuse de mise en oeuvre de l'invention, les algorithmes utilisent les déviations d'amplitude et de phase comme des opérateurs permettant de bâtir des filtres et confirmer la présence ou l'absence des défauts.

Les exemples qui suivent, donnés dans un but uniquement illustratif, illustrent les avantages de la présente invention :

### Exemples :

Différents défauts ont été observés ou simulés sur un matelas de laine de roche en sortie d'une ligne de fabrication. Pour différentes épaisseurs et densités du matelas de fibres, les défauts ont été caractérisés en utilisant le dispositif tel que décrit précédemment en relation avec la figure 2. On parle d'observation lorsque les défauts sont directement issus du procédé de fibrage et de simulation lorsque les défauts sont intentionnellement introduits dans l'épaisseur du matelas de laine de roche, après le fibrage et avant le passage du matelas sous le dispositif de détection.

Le dispositif a été installé sur une chaîne de fabrication de panneau de laine de roche dans la configuration décrite en figure 1.
La fréquence du signal micro-onde a été fixée dans tous les exemples à 9,4 MHz, la puissance totale rayonnée par les émetteurs 105 étant de 0,3 Watt. La distance entre deux émetteurs ou capteurs 105 est de 10 mm. La distance entre deux récepteurs ou capteurs 106 est également de 10 mm. De la manière la plus simple, le traitement informatique du signal comprend une comparaison de la variation maximale de la mesure donnée par une sonde en phase et en amplitude au passage du défaut, par rapport à une valeur moyenne obtenue sur un échantillon sans défaut ou alternativement par rapport à une moyenne des mesures obtenues pour ladite sonde, lors du défilement du tapis de fibres. Les valeurs maximales des déviations de phase et d'amplitude, pour chaque défaut, sont répertoriées dans le tableau 1, en valeur absolue.

Lorsqu'un défaut est observé par le dispositif selon l'invention, la partie du matelas contenant le défaut a été prélevée et analysée. Dans tous les cas, l'analyse a effectivement révélé la présence d'un défaut dans l'épaisseur du matelas aux endroits exacts détectés par le dispositif, que celui-ci soit simplement observé, c'est-à-dire issu du procédé de fibrage lui-même ou simulé, c'est-à-dire introduit intentionnellement après fibrage.

L'ensemble des données est reporté dans le tableau 1.

| Caractéristiques du matelas de laine de roche | Nature du défaut | Origine du défaut | Variation de phase | Variation d'amplitude |
|---|---|---|---|---|
| Epaisseur 30 mm Densité 110 kg/m³ | Point humide | observé | 80° | 70 à 80 % |
| | Point humide | simulé (injection par une aiguille de 5 ml d'eau au centre du matelas) | 40° | 30% |
| | Point chaud | Observé (Roche en fusion) | 60° | 60% |
| | Morceau de verre | simulé (ajout d'un morceau de verre fondu de 1 cm sur le matelas) | 20° | 25% |
| | Résidus provenant de la paroi du four | simulé (morceaux de 3 cm de diamètre incorporé dans le matelas) | 30° | 30% |
| Epaisseur 100 mm Densité 100 kg/m³ | Point humide | observé | 40° | 50% |
| Epaisseur 100 mm Densité 40 kg/m³ | Variation de densité | simulé (augmentation locale à 50 kg/m³) | 10 à 15° | 10% |
| | Résidus provenant de la paroi du four | Simulé (morceau de réfractaire de 6 cm de diamètre | 30° | 50% |

## Revendications

1. Méthode de détection de défauts localisés, par exemple du type points chauds ou points humides, présents dans un matelas de fibres minérales liés par un liant, comprenant l'utilisation d'un rayonnement micro-ondes de fréquence comprise entre 1 et 50 GHz, la puissance totale de l'onde électromagnétique émise étant comprise entre 0,1 et 5 Watts, **caractérisée en ce que** le rayonnement micro-onde incident est mis en oeuvre au moyen d'un réseau de dispositifs émetteurs des micro-ondes, disposés transversalement en regard d'une face du matelas et orientés en direction dudit matelas et dans laquelle le signal, après traversée dudit matelas, est recueilli par un réseau de sondes réceptrices ou de capteurs disposés transversalement en regard de la face opposée du matelas, et **en ce que** la détection est effectuée à la sortie des moyens de réticulation dudit liant.

2. Méthode selon la revendication 1, dans laquelle ledit rayonnement micro-onde est recueilli et analysé après avoir traversé ledit matelas de fibres minérales.

3. Méthode selon la revendication 1 ou 2 dans laquelle la présence desdits défauts est **caractérisée par** une mesure du décalage de la phase du rayonnement micro-ondes et/ou de la modification de l'amplitude dudit rayonnement, lorsque celui-ci traverse le matelas de fibres minérales.

4. Méthode selon l'une des revendications précédentes, dans laquelle le matelas défile entre le réseau de dispositifs émetteurs et le réseau de sondes réceptrices, la détection desdits défauts étant effectuée en continu sur toute la largeur du matelas de fibres.

5. Méthode selon la revendication 4, dans laquelle le matelas de fibres défile sur un convoyeur à rouleau, les dispositifs émetteurs et les capteurs étant disposés respectivement au dessus et en dessous du convoyeur.

6. Méthode selon l'une des revendications précédentes, dans laquelle l'écartement entre les capteurs est choisi en fonction de la taille desdits défauts.

7. Méthode selon l'une des revendications précédentes, dans laquelle l'écartement entre les capteurs est compris entre 1 et 100 mm, de préférence entre 5 et 20 mm.

8. Méthode selon l'une des revendications précédentes, dans laquelle l'épaisseur du matelas de fibres minérales est comprise entre 30 et 400 mm et dans laquelle la densité du matelas de fibres minérales est comprise entre 6 et 220 kg/m³.

9. Méthode selon l'une des revendications précédentes, dans laquelle le rayonnement micro-ondes présente une fréquence comprise entre 5 et 10 GHz.

10. Méthode selon l'une des revendications précédentes, dans laquelle la puissance totale de l'onde électromagnétique émise est de l'ordre de 1 Watt.

11. Méthode selon l'une des revendications précédentes, dans laquelle ladite méthode est mise en oeuvre par un dispositif de détection comprenant des moyens de génération d'un rayonnement micro-ondes (101, 108, 103, 105) de fréquence comprise entre 1 et 50 GHz, de préférence entre 5 et 10 GHz, la puissance totale de l'onde électromagnétique émise étant comprise entre 0,1 et 5 Watts, de préférence de l'ordre de ou inférieur à 1 Watt et des moyens de détection de l'onde électromagnétique modulée (106, 104, 109, 101), après traversée du matelas de fibres (10), **caractérisé en ce que** les moyens de génération comprennent un réseau de dispositifs émetteurs de micro-ondes (105) disposés de manière à pouvoir être orientés transversalement selon une face du matelas et en direction dudit matelas et dans lequel les moyens de détection comprennent un réseau de capteurs (106) disposés transversalement en regard de la face opposée du matelas de fibres.

12. Méthode selon la revendication 11 dans laquelle ledit dispositif comprend en outre des moyens de traitement et de visualisation du signal (102).

13. Installation de fabrication d'un matelas continu de fibres minérales liées par un liant, du type laine de roche ou laine de verre, comprenant des moyens de fibrage (1) des fibres minérales, des moyens de synthèse et d'aspersion du liant (7), des moyens de collecte (2), des moyens de convoyage (9) et des moyens de réticulation du liant(3), ladite installation **se caractérisant en ce qu'**elle comprend en outre un dispositif de détection (4, 6) selon l'une des revendications 11 ou 12, disposé à la sortie des moyens de réticulation.

14. Installation selon la revendication 13, dans laquelle le dispositif de détection est couplé à des moyens de régulation (15) d'au moins un paramètre choisi dans le groupe constitué par la composition du liant, la force de l'aspiration, la température de réticulation des fibres, le temps de séjour dans les moyens de réticulation.

15. Installation selon la revendication 13 ou 14, dans laquelle le dispositif de détection est couplé à des moyens de régulation (15') d'un organe (5) disposé en aval du dispositif d'analyse micro-ondes (4), ledit organe (5) étant configuré pour isoler et/ou déclasser les zones du matelas incluant les défauts ou pour effectuer un marquage desdites zones, par exemple en vue d'une découpe ou d'un tri ultérieur.

## Patentansprüche

1. Verfahren zur Erfassung von lokalisierten Mängeln, beispielsweise vom Typ heiße oder feuchte Punkte, die in einer Matte aus Mineralfasern vorliegen, die mit einem Bindemittel gebunden sind,
das die Verwendung einer Mikrowellenstrahlung mit einer Frequenz zwischen 1 und 50 GHz umfasst, wobei die Gesamtleistung der ausgesendeten elektromagnetischen Welle zwischen 0,1 und 5 Watt liegt, **gekennzeichnet dadurch, dass** die inzidierende Mikrowellenstrahlung mithilfe eines Netzes aus Mikrowellenstrahlungsgeräten ausgesendet wird, die transversal gegenüber einer Seite der Matte angeordnet und in Richtung der genannten Matte ausgerichtet sind, und bei der das Signal nach Passieren der genannten Matte von einem Netz aus Empfangssonden oder Messfühlern aufgenommen wird, die transversal im Verhältnis zur entgegengesetzten Seite der Matte angeordnet sind, und **gekennzeichnet dadurch, dass** die Erfassung am Ausgang der Vernetzungsanlagen des genannten Bindemittels erfolgt.

2. Verfahren nach Anspruch 1, bei dem die genannte Mikrowellenstrahlung erfasst und analysiert wird, nachdem sie die genannte Mineralfasermatte passiert hat.

3. Verfahren nach Anspruch 1 und 2, bei dem das Vorliegen der genannten Mängel durch eine Messung der Phasenverschiebung der Mikrowellenstrahlung und/oder die Veränderung der Amplitude der genannten Strahlung charakterisiert ist, wenn diese die Mineralfasermatte passiert.

4. Verfahren nach einem der vorangehenden Ansprüche, bei dem die Matte durch das Netz aus Bestrahlungsgeräten und das Netz aus Empfangssonden vorbeizieht, wobei die Erfassung der genannten Mängel fortlaufend über die gesamte Länge der Fasermatte erfolgt.

5. Verfahren nach Anspruch 4, bei dem die Fasermatte auf einer Rollenbahn vorbeizieht, wobei die Bestrahlungsgeräte und Messfühler jeweils oberhalb und unterhalb der Rollenbahn angebracht sind.

6. Verfahren nach einem der vorangehenden Ansprüche, bei dem der Abstand zwischen den Messfühlern im Verhältnis zur Größe der genannten Mängel gewählt wird.

7. Verfahren nach einem der vorangehenden Ansprüche, bei dem der Abstand zwischen den Messfühlern zwischen 1 und 100 mm, vorzugsweise zwischen 5 und 20 mm, liegt.

8. Verfahren nach einem der vorangehenden Ansprüche, bei dem die Stärke der Mineralfasermatte zwischen 30 und 400 mm liegt und bei der die Dichte der Mineralfasermatte zwischen 6 und 220 kg/m³ liegt.

9. Verfahren nach einem der vorangehenden Ansprüche, bei dem die Mikrowellenstrahlung eine Frequenz zwischen 5 und 10 GHz aufweist.

10. Verfahren nach einem der vorangehenden Ansprüche, bei dem die Gesamtleistung der ausgesendeten elektromagnetischen Welle in einer Größenordnung von 1 Watt liegt.

11. Verfahren nach einem der vorangehenden Ansprüche, bei dem das genannte Verfahren mithilfe eines Erfassungsgeräts realisiert wird, das aus Geräten zur Erzeugung einer Mikrowellenstrahlung (101, 108, 103, 105) mit einer Frequenz zwischen 1 und 50 GHz, vorzugsweise zwischen 5 und 10 GHz, besteht, wobei die Gesamtleistung der ausgesendeten elektromagnetischen Welle zwischen 0,1 und 5 Watt, vorzugsweise in einer Größenordnung von 1 Watt oder kleiner liegt, sowie aus Geräten zur Erfassung der modulierten elektromagnetischen Welle (106, 104, 109, 101) nach der Passage der Fasermatte (10), **gekennzeichnet dadurch, dass** die Geräte zur Generierung aus einem Netz aus Mikrowellenstrahlungsgeräten (105) bestehen, die so angeordnet sind, dass sie transversal zu einer Seite der Matte sowie in Richtung der genannten Matte ausgerichtet werden können, und **gekennzeichnet dadurch, dass** die Erfassungsgeräte ein Netz aus Messfühlern (106) umfassen, die transversal im Verhältnis zur entgegengesetzten Seite der Fasermatte angeordnet sind.

12. Verfahren nach Anspruch 11, bei dem das genannte Gerät zusätzlich Aufbereitungs- und Anzeigegeräte für das Signal (102) umfasst.

13. Produktionsanlage für eine durchgehende Matte aus Mineralfasern, die durch ein Bindemittel gebunden sind, vom Typ Gesteinsfaser oder Glaswolle, bestehend aus Faserziehmaschinen (1) für Mineralfasern, Synthese- und Sprühanlagen für das Bindemittel (7), Sammelvorrichtungen (2), Förderanlagen (9) sowie Vernetzungsanlagen für das Bindemittel (3), wobei die genannte Produktionsanlage **dadurch gekennzeichnet ist, dass** sie zusätzlich ein Erfassungsgerät (4, 6) nach einem der Ansprüche 11 oder 12 beinhaltet, das am Ausgang der Vernetzungsanlagen angeordnet ist.

14. Anlage nach Anspruch 13, bei der das Erfassungsgerät an Regelvorrichtungen (15) mindestens eines Parameters innerhalb der Gruppe gekoppelt ist, der aus der Zusammensetzung des Bindemittels, der Vernetzungstemperatur oder der Verweildauer in den Vernetzungsmitteln gewählt wird.

15. Anlage nach Anspruch 13 oder 14, bei der das Erfassungsgerät an Regelvorrichtungen (15') einer Komponente (5) gekoppelt ist, die dem Mikrowellenanalysegerät (4) nachgeschaltet ist, wobei die genannte Komponente (5) so konfiguriert ist, dass sie die Bereiche der Matte, die die Mängel enthalten, isoliert und/oder abstuft oder eine Markierung an den genannten Bereichen vornimmt, z. B. zwecks Zuschnitts oder späterer Aussortierung.

## Claims

1. A method for detecting localized defects, for example of the hot spot or wet spot type, present in a mineral fiber mat bound by a binder, comprising the use of microwave radiation with a frequency between 1 and 50 GHz, the total power of the electromagnetic wave emitted being between 0.1 and 5 watts, **characterized in that** the incident microwave radiation is implemented by means of an array of microwave emitter devices, positioned transversely facing one side of the mat and oriented in the direction of said mat, and wherein, after passing through said mat, the signal is collected by an array of receiver probes or sensors positioned transversely facing the opposite side of the mat and **in that** said detection is made at the outlet of the crosslinking means of said binder.

2. The method as claimed in claim 1, wherein said microwave radiation is collected and analyzed after having passed through said mineral fiber mat.

3. The method as claimed in claim 1 or 2, wherein the presence of said defects is **characterized by** a measurement of the phase shift of microwave radiation and/or modification of the amplitude of said radiation, when this radiation passes through the mineral fiber mat.

4. The method as claimed in claim 3, wherein the fiber mat passes between the array of transmitter devices and the array of receiver probes, the detection of said defects being carried out continuously over the entire width of the fiber mat.

5. The method as claimed in claim 4, wherein the fiber mat passes on a roller conveyor, the transmitter devices and the sensors being positioned above and below the conveyor respectively.

6. The method as claimed in one of the preceding claims, wherein the gap between the sensors is chosen depending on the size of said defects.

7. The method as claimed in one of the preceding claims, wherein the gap between the sensors is between 1 and 100 mm, preferably between 5 and 20 mm.

8. The method as claimed in one of the preceding claims, wherein the thickness of the mineral fiber mat is between 30 and 400 mm and wherein the density of the mineral fiber mat is between 6 and 220 kg/m³.

9. The method as claimed in one of the preceding claims, wherein the microwave radiation has a frequency between 5 and 10 GHz.

10. The method as claimed in one of the preceding claims, wherein the total power of the electromagnetic wave emitted is about 1 watt.

11. The method as claimed in one of the preceding claims, wherein said method is implemented by a detection device comprising means for generating microwave radiation (101, 108, 103, 105) with a frequency of between 1 and 50 GHz, preferably between 5 and 10 GHz, the total power of the electromagnetic wave emitted being between 0.1 and 5 watts, preferably around or less than 1 watt, and means for detecting the modulated electromagnetic wave (106, 104, 109, 101) after passing through the fiber mat (10), **characterized in that** the generation means include an array of microwave transmitter devices (105) positioned so as to be able to be oriented transversely along one side of the mat and in the direction of said mat, and wherein the detection means comprise an array of sensors (106) positioned transversely facing the opposite side of the fiber mat.

12. The method according to claim 11, wherein said device further comprises means for processing and displaying the signal (102).

13. An installation for the manufacture of a continuous mineral fiber mat bound by a binder, of the rock wool or glass wool type, comprising means for fiberizing (1) the mineral fibers, means for synthesizing and spraying the binder (7), and means for collecting (2), means for conveying (9) and means for crosslinking the binder (3), said installation furthermore comprising a detection device (4, 6) according to one of claims 11 or 12, positioned at the outlet of the crosslinking means.

14. The installation as claimed in claim 13, wherein the detection device is coupled to means (15) for regulating at least one parameter chosen from the group consisting of the composition of the binder, the suction force, the crosslinking temperature of the fibers, and the residence time in the crosslinking means.

15. The installation as claimed in claim 13 or 14, wherein the detection device is coupled to means (15') for regulating a piece of equipment (5) positioned downstream of the microwave analysis device (4), said piece of equipment (5) being configured to isolate and/or downgrade the areas of the mat that include the defects or to mark said areas, for example with a view to subsequent sorting or cutting.
